**Europäisches Patentamt**

**European Patent Office**

**Office européen des brevets**

(19)

(11) Veröffentlichungsnummer: **0 531 562 A1**

# (12) EUROPÄISCHE PATENTANMELDUNG

(21) Anmeldenummer: **91115336.9**

(51) Int. Cl.5: **C12N 5/00**

(22) Anmeldetag: **11.09.91**

(43) Veröffentlichungstag der Anmeldung:
**17.03.93 Patentblatt 93/11**

(84) Benannte Vertragsstaaten:
**DE**

(71) Anmelder: **Doerr, Hans-Wilhelm, Prof. Dr. med.
Breitseeweg 21
W-6072 Dreieich-Buchschlag(DE)**

(72) Erfinder: **Cinatl, Jaroslaw, Dr.
Ludwig-Rehn-Strasse 16
W-6000 Frankfurt/Main 70(DE)**
Erfinder: **Cinatl, Jindrich, Dr.
Johann-Sebastian-Bach-Strasse 17
W-6053 Obertshausen(DE)**

(74) Vertreter: **Laudien, Dieter, Dr. et al
Boehringer Ingelheim GmbH, Abteilung
Patente
W-6507 Ingelheim am Rhein (DE)**

(54) **Kultivierung von Säugetierzellen.**

(57) PVF und/oder PVB wird für die Kulturoberfläche in der Züchtung adhärenter Zellen in proteinfreiem Medium verwendet.

EP 0 531 562 A1

Die Erfindung betrifft die Kultivierung von Säugetierzellen, insbesondere die Kultivierung in serumfreiem Medium. Sie ist z.B. für die Kultivierung von adhärenten Zellinien einschließlich zur Züchtung von Viren auf infizierten adhärenten Zellen geeignet.

Die Kultivierung von Säugetierzellen in einem chemisch definierten Medium ohne Proteininhalt ist seit mehr als 30 Jahren bekannt [1, 2], ebenso verschiedene Zellinien, die in proteinfreiem Medium etabliert worden sind [3-13]. Medien, die als Handelsprodukte zur Verfügung stehen, um das Wachstum von Zellen in Abwesenheit von fremden Proteinen zu ermöglichen, sind erst kürzlich entwickelt worden [14, 15].

Diese bekannten proteinfreien Medien sind in erster Linie für die Züchtung von kontinuierlichen Zellinien in einer Suspension wie z.B. Hybridoma geeignet [10, 14, 15], andererseits häufig nicht geeignet, um das Zellwachstum in Monolayerkulturen zu fördern [23].

Dabei wurde dargestellt, daß die Eigenschaften der Kulturoberflächen das Wachstum der Monolayerkulturen in proteinfreiem Medium signifikant beeinflussen [6, 16, 17]. Rappaport et al. haben gefunden, daß die Adhäsion und das Wachstum von Zellen abhängig von einer kritischen Anzahl von negativen Ladungen auf die Oberflächen sind. Sie haben nach einem besonderen Verfahren Glasflaschen mit Alkali behandelt, dadurch wurde die Kultivierung verschiedener Zellinien ohne vorhergehende Anpassung in einem proteinfreien Medium ermöglicht [24]. Diese Technik zur Vorbereitung der Kulturoberfläche ist allerdings umständlich und auf das Material Glas beschränkt. Außerdem wurden unterschiedliche Kunststoffoberflächen von verschiedenen Lieferanten in Assays unter Verwendung von Zellen in serumhaltigen Medien und anderen Proteinzusätzen geprüft. Die Ergebnisse dieser Untersuchung zeigten, daß für das Wachstum der Zellen in proteinfreiem Medium eine andere Qualität der Kulturoberfläche erforderlich ist als für das Wachstum der Zellen in serumhaltigem Medium.

Daß die Eigenschaften der Oberflächen aus Glas oder Kunststoff einen signifikanten Einfluß auf das Wachstum der Einzelschichtzellen in einem proteinfreiem Medium haben, ist also seit Jahren bekannt [6, 16, 17]. Der Literatur ist daher zu entnehmen, daß nicht nur die Zusammensetzung des Mediums sondern auch die Eigenschaften der Kulturoberflächen optimiert werden müssen, um ein ausreichendes Wachstum der Zellen in einem solchen Medium zu erreichen.

Polyvinylformal (PVF) ist erstmalig von Barski und Maurin als Kulturoberfläche für mehrere Zelltypen in Medien, die Serum, Embroyalextrakt und Tyrodes Salzlösung enthielten, benutzt worden. Andere Autoren verwendeten PVF vor einiger Zeit zur Züchtung von verschiedenen Zellinien in Medien mit Protein-Zusatz [26-28].

Im Handel erhältliche Wegwerfartikel aus Kunststoff einschließlich Polysterin (PS) besitzen meist eine negative Oberflächenladung. Maroudas hat nachgewiesen, daß für die Ausbreitung der Zellen eine Dichte der negativen Ladung von $2\text{-}10 \times 10^{14}$ per $cm^2$ am günstigsten ist [18, 19], z.B. hat PS-TC eine negative Ladung von $4\text{-}7 \times 10^{14}$ per $cm^2$. Andererseits hat PVF eine negative Ladung von nur $0.4\text{-}0.8 \times 10^{14}$ per $cm^2$.

Aufgabe der Erfindung ist es, eine neue Kulturoberfläche zur Verfügung zu stellen, in der verschiedene Zellinien, vorzugsweise als kontinuierliche Monolayer, in einem proteinfreien Medium kultiviert werden können.

Diese Aufgabe wird überraschenderweise durch die Verwendung von PVF und/oder PVB als Zellsubstrat erfüllt.

Gegenstand der Erfindung ist daher ein Verfahren zur Kultivierung von adhärenten Zellen in proteinfreiem Medium, dadurch gekennzeichnet, daß man Kulturoberflächen aus Polyvinylformal und/oder Polyvinylbutyral verwendet.

Polyvinylformal (PVF) und Polyvinylbutyral (PVB) sind bekannte Polymere der Gruppe "Polyvinylacetals", die durch die Reaktion von Aldehyde (Formaldehyde oder Butyraldehyde) und Polyvinylalkohol entstehen können. Bekanntlich kann man durch Anwendung von entsprechenden Mengen der Ausgangsmonomere PVF und PVB mit verschiedenen gezielten Inhalten von z.B. Hydroxylgruppen erhalten, z.B. enthalten die Handelsprodukte Butvar(R)B-98 (Monsanto Company, Texas) 20% Hydroxyl als Polyvinylalkohol und Butvar(R)B-76 13% Hydroxyl unter den gleichen Voraussetzungen; verschiedene Formvar(R)-Harnstoffe wie z.B. Formvar(R) 5/95E und 15/95E enthalten nur 6.5% Hydroxy als Polyvinylalkohol. Butvar(R) ist damit hydrophiler als Formvar(R). PVF und PVB können allein oder als Mischungen verwendet werden.

Vorzugsweise werden die Zellen als kontinuierliche Monolayerkulturen kultiviert.

Das Verfahren der Erfindung ist besonders für die Kultivierung von Vero-Zellen geeignet.

Als proteinfreies Medium kann man z.B. ein 1:1 modifiziertes DMEM/F12-Medium (ergänzt mit Spurenelementen, C-Vitamin Phosphat und Glutamindipeptid), Ham'sF10, Ham'sF12, $\alpha$-MEM oder DMEM/F12 ohne Spurenelemente und C-Vitamine verwenden.

Bevorzugt wird die oben erwähnte modifizierte Mischung aus DMEM F12. Für den Fachmann ist es einfach, die verschiedenen zur Verfügung stehenden Medien in diesen Verfahren analog der Beispiele zu testen, um die passenden Medien zu identifizieren.

Vorzugsweise werden die Zellen in einer ursprünglichen Zelldichte von mindestens $2.0 \times 10^4$ Zellen/cm$^2$ inkubiert.

Weiterer Gegenstand der Erfindung zur Kultivierung der Zellen in proteinfreiem Medium ist die Verwendung eines Kultivierungsreaktors, der eine Kulturoberfläche aus PVF und/oder PVB enthält.

Als Kulturoberfläche, beschichtet mit PVF und/oder PVB können verschiedene Füllungskörper wie z.B. Sattel, Stahl-Spiralen, Glas- bzw. Keramikbeads usw. dienen.

Obwohl das Kulturgefäß oder der Füllkörper ganz aus PVF bestehen können, wird normalerweise eine dünne Schicht aus PVF auf eine Grundstruktur aus Metall, Glas oder Kunststoff, z.B. nach bekannten Methoden, abgelagert. Vorzugsweise wird die beschichtete Oberfläche durch ein Verfahren hergestellt, mit dem man die Oberfläche einer Grundstruktur in Kontakt mit einer Lösung von PVF bringt und das Lösungsmittel verdampft oder verdampfen läßt.

Das Verfahren der Erfindung eignet sich auch zur Herstellung von biologischen Substanzen durch Kultivierung von Zellen, die die Substanz als endogenes Protein oder als rekombinantes Protein produzieren. Zu diesem Zweck kann z.B. ein durch ein für ein fremdes Protein kodierendes Gen transformierte Säugetierzelle auf der PVF und/oder PVB-Kulturoberfäche im proteinfreien Medium kultiviert werden und nach Ende der Kultivierung das Protein durch an sich bekannte Methoden isoliert und gereinigt werden. Als biologische Substanzen kommen z.B. t-PA, EPO, hGH, ICAM-1, humane Lungensurfactant-Proteine wie z.B. SP-B, SP-C usw. in Frage.

Fig. 1 zeigt die stilisierten Formeln von PVF mit Hydroxyl Gruppen, Acetat-Gruppen und Formal-Gruppen, die willkürlich entlang der Moleküle verteilt sind. Die Formar-Verbindung von PVF enthält 5-5.6% Polyvinylalkohol, 9.5-13% Polyvinylacetat und 82% Polyvinylformal.

Fig. 2 zeigt die Wachstumskurven von Vero-Zellen in MEM, mit 10% FBS (—x—) ergänzt, und von Vero-Zellen in dem proteinfreien Medium auf PS-TC- (—△—), auf Poly-D-Lysine- (—*—) und auf PVF-Oberflächen (—□—).

Fig. 3 zeigt das morphologische Erscheinungsbild von Vero-Zellen im proteinfreien Medium auf PS-TC-Oberflächen (A), Poly-D-Lysine-Oberflächen (B) und PVF-Oberflächen (C). 9 Tage nach der Beimpfungphase unter dem Kontrast-Mikroskop.

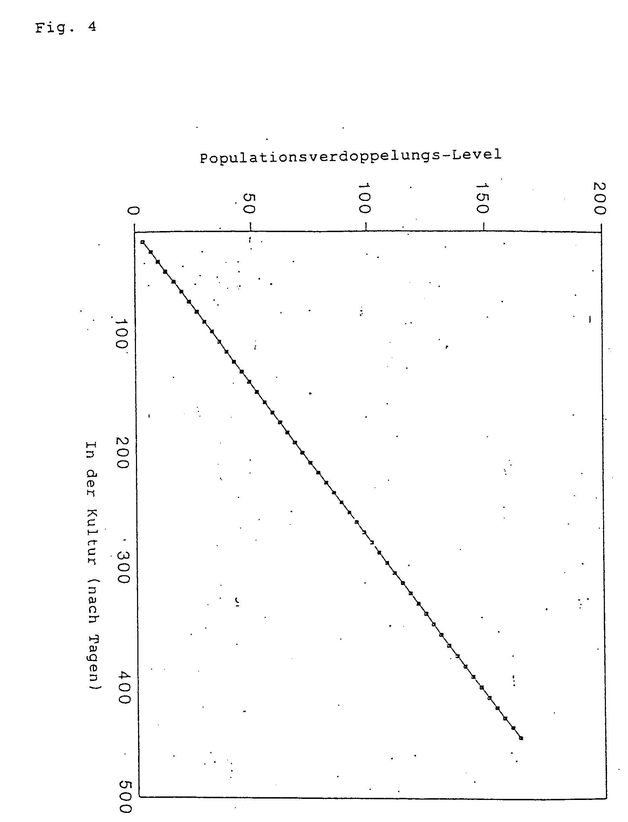

Fig. 4 zeigt die Langzeitkultivierung von Vero-Zellen in proteinfreiem Medium an PVF-beschichteten Oberflächen. Die Zellen wurden in Intervallen von 9 Tagen unter Verwendung einer Kollagenase-Dispase-Lösung subkultiviert und in einer Dichte von $2.5 \times 10^4$ Zellen per cm$^2$ eingesät.

Fig. 5 zeigt das morphologische Erscheinungsbild von Vero-Zellen nach 40 Passagen im proteinfreien Medium an PVF-beschichteten Oberflächen. Die Zellen wurden mit May-Grünwald-Giemsa gefärbt.

Beispiel 1

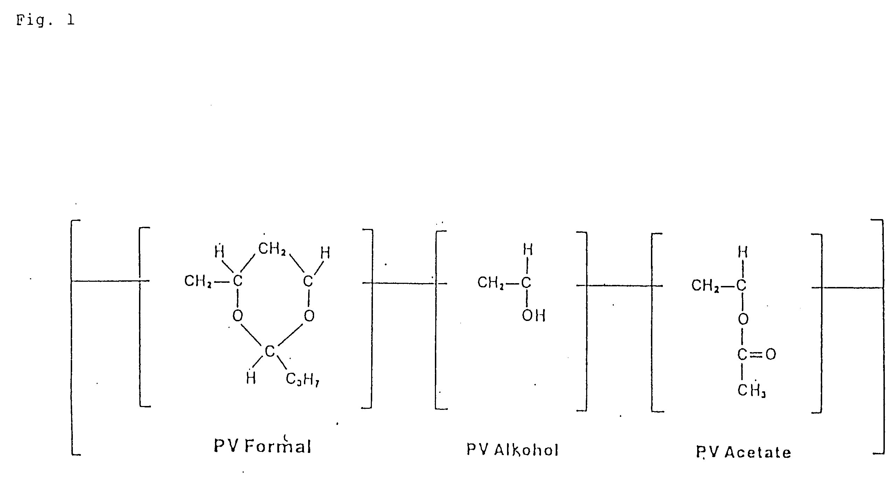

Eine Polyvinylformal-Lösung erhält man durch die Auflösung von pulverisiertem Polyvinylformal (PVF, Fig. 1) (Formvar$^{(R)}$; Monsanto Company, Texas, USA) in Essigsäure in verschiedenen Konzentrationen von 1 bis 20 mg/ml. Die resultierende Lösung wurde in Mengen von 5 ml oder 2 ml auf die Kulturoberfläche von jeweils 25 cm$^2$ PS-TC-Kolben (Falcon, Heidelberg, BRD, und Greiner, Nürtingen, BRD) und Schalen (Greiner, Nürtingen, BRD) gegeben; nach 2 Minuten wurde die Lösung abgegossen. Die Kolben wurden 24 Stunden lang in einen Laminar Flowhood gestellt, um den Rest der Essigsäure verdampfen zu lassen. Danach wurden die Kolben mit doppeltdestilliertem Wasser gewaschen und entweder sofort zur Zellkultur verwendet oder bis zu 3 Monate nach ihrer Vorbereitung.

Die negative Ladungsdichtigkeit der Oberflächen der Gefäße wurde mittels einer kationischen Farbstoffbindung bestimmt (wie bei Maroudas beschrieben [10, 19]).

Es wurde angenommen, daß die Anzahl der Moleküle, die an die Oberfläche gebunden sind, mit der absoluten Anzahl der festen negativen Ladungen identisch sind.

Als Zellinien wurden murine NCTC Klon 929L und Swiss 3TC, syrische Hamster BHK-21 Klon 13, chinesische Hamster CHO-K1, Schweine PK-15, Hunde MDCK, Affen Vero und LLC MK2, humanes A431 und HeLa-Zellinien von der American Type Culture Collection verwendet.

Stockkulturen dieser Zellen, mit Ausnahme der CHO-Zellen, wurden in MEM enthaltendem 5-10% Fetal Bovine Serum (FBS) vermehrt. Die CHO-Zellen wurden in Ham's F12, 10% FBS enthaltend, gezüchtet.

Alle Zellen wurden in Kulturflaschen mit einer Kulturoberfläche von 75 cm$^2$ in einem $CO_2$ Inkubator (37°C, 5% $CO_2$) kultiviert. Die Zellen wurden routinemäßig kontrolliert, um Mycoplasma mittels der Hoechst 33258 DNA Staining-Methode zu identifizieren. Alle Färbungen waren negativ.

Das proteinfreie Medium war eine modifizierte 1:1-Mischung aus DMEM-F12, enthaltend 15 mM HEPES und 1.2 g/l Natrium bicarbonat (Tabelle 1). Die Verwendung von Glycyl-L Glutamine anstelle von L-Glutamine hatte eine erhöhte Konzentration von Glycin in dem Medium zur Folge. Diese erhöhte Konzentration von Glycin hatte aber keinen Einfluß auf die Vermehrung der verschiedenen Zellen.

Um die proteinfreien Zellkulturen in Gang zu setzen, wurden die konfluenten Kulturen dreimal mit PBS gewaschen und von der Kulturoberfläche unter Verwendung einer 2:1 Mischung aus Kollagenase H 0.25% und Dispase 0.25% in PSB freigesetzt. Die freigesetzten Zellen wurden dreimal mit PBS gewaschen und in proteinfreiem Medium innokuliert und anschließend im $CO_2$-Inkubator (37°C, 5% $CO_2$) inkubiert.

Um das Zellwachstum und die Adhäsion zu messen, wurden die Zellen dreimal mit PBS gewaschen und zwar in unterschiedlichen Zeitabständen nach der Innokulation, von der Kulturoberfläche mittels Trypsin 0.1% in PBS freigesetzt und die vitalen Zellen mittels eines Hemocytometers ausgezählt.

Die Lebensfähigkeit der Zellen wurde mittels der Farbstoff-Exclusion-Methode ermittelt, als Farbstoff wurde 0.5% Trypan-Blau-Lösung verwendet.

Die Sättigungsdichte der Zellpopulation stellt die maximale Anzahl der Zellen dar, die unter spezifischen Kulturbedingungen [22] erreicht werden kann. Der Wert der Populations-Verdoppelung (PDL) wurde aus der ursprünglichen Zahl, die als Impfung benutzt wurde (Ni), und der Zahl der geernteten Zellen (Nf) unter Benutzung der Formel PDL = 3.32 log (Nf/Ni) [22] ermittelt.

Beispiel 2

Polioviren der Typen 1 bis 3, klinische Isolate, und Adenoviren der Typen 2 und 5 wurden aus adenoidem Gewebe isoliert. Die Identität der Viren wurde mittels der Serum-Neutralisations-Untersuchung unter Verwendung der standardisierten Polioviren der Typen 1 bis 3- und Adenoviren der Typen 2 und 5-Antisera festgestellt. Alle Viren wurden in Verozellen propagiert und titriert, die in MEM, 2% FBS enthaltend, gehalten wurden.

Um eine Kontaminierung des proteinfreien Mediums durch Serumproteinen aus dem obigen Virenstamm zu vermeiden, wurden nur intrazellulare Viren der infizierten Kulturen in der folgenden Untersuchung verwendet.

Die infizierten Zellen wurden von der Kulturoberfläche gekratzt, dreimal mit PBS gewaschen und jedesmal in neuem proteinfreiem Medium suspendiert. Die Zellen wurden danach mittels Utraschall lysiert, zentrifugiert (1200 xg) und die Viren enthaltenden Überstände wurden bei -70°C gefroren.

Konfluente Kulturen der Vero wurden in 35 mm Kulturschalen, die auf PVF-Oberflächen in proteinfreiem Medium kultiviert wurden, mit 0.2 ml Virensuspension, die Polioviren mit MOI 0.01 PFU/Zellen enthalten, gegeben. Nach einer Stunde bei 37°C wurde das Inokulum entfernt, die Zellen mit PBS gewaschen und 2 ml proteinfreies DMEM/F12 zugesetzt. Nach 3 Tagen wurden die Überstände gesammelt und Virustitration durch Plaqueassay vorgenommen. Zu diesem Zweck wurden die Überstände der infizierten Kulturen verdünnt (in zweifachen Schritten).

Dazu wurden konfluente Verozellkulturen mit PBS gewaschen und die jeweilige Verdünnung mit 0.2 ml Virussuspension geimpft. Nach 1 Stunde Inkubation wurde das Inokulum entfernt und die inokulierten Zellen mit PBS gewaschen. Die so infizierten Verozellen wurden zur Titerbestimmung 4 Tage (Polio) oder 7 Tage (Adeno) mit 2 ml 2%iger Carboxymethylzellulose in MEM + 2% FCS bei 37°C ($CO_2$-Inkubator) gehalten. Pro Verdünnung wurden 3 Kulturschalen angesetzt. Nach der entsprechenden Inkubationszeit wurden die Zellen gewaschen (PBS) und mit 0.2% Kristallviolett gefärbt und die Titer bestimmt.

Ergebnis:

In Tabelle 2 wird die Wirkung der PVF-Oberfläche auf das Wachstum von zehn kontinuierlichen Zellinien in proteinfreiem DMEM-F12 mit der Wirkung von PS-TC oder Poly-D-Lysine verglichen.

Alle Zellen wurden bei einer Dichte von $2x10^4$ Zellen/$cm^2$ eingesät. Die Ergebnisse entsprachen der Sättigungsdichte der Zellen in den verschiedenen Zeitabständen zwischen 5 und 9 Tagen.

3T6 war die einzige Zellinie, die minimal auf der PVF anhaftete und keine wesentliche Ausbreitung zeigte, während 3T6-Zellen auf PS-TC oder Poly-D-Lysine direkt nach ihrer Adhäsion eine Anfangsproliferation aufweisen.

Sechs Zellinien einschließlich der NCTC Klon 929L, BHK, CHO-K1, PK 15, A431 und HeLa weisen die gleiche Ausbreitung auf PVF und auf den handelsüblichen PS-Flaschen auf. In den meisten Fällen konnte man das Zellwachstum durch die Behandlung einer Oberfläche von Poly-D-Lysine mit PS-TC steigern, eine Konfluenz von mehr als 75% kann damit nicht erreicht werden. Zwei Zellinien einschließlich MDCK und LLC-MK2 konnten sich wesentlich besser auf PVF als auf PS-TC ausbreiten. Die Ausbreitung von MDCK auf PVF wurde im Vergleich zu den Zellen auf der aus Poly-D-Lysine bestehenden Oberfläche etwas gesteigert. Diese Zellen konnten jedoch auch weder eine konfluente Zellschicht noch ein kontinuierliches Wachstum erreichen. Demgegenüber wuchsen die Vero-Zellen viel besser auf PVF als auf aus PS-TC oder

4

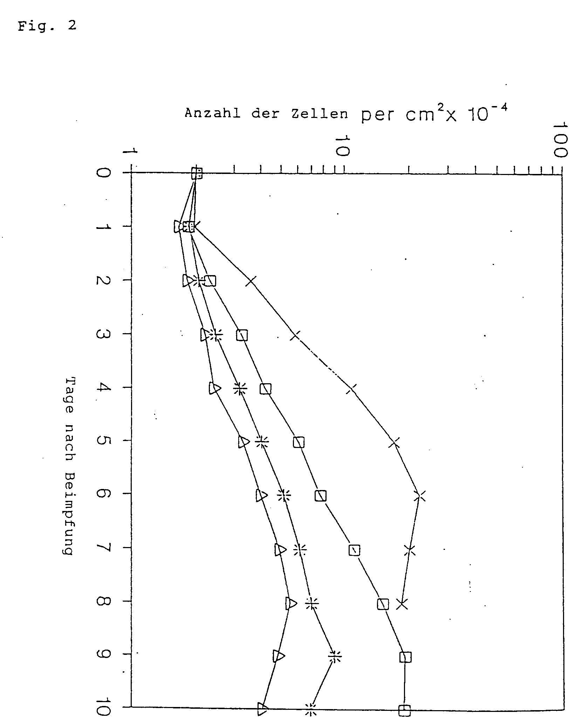

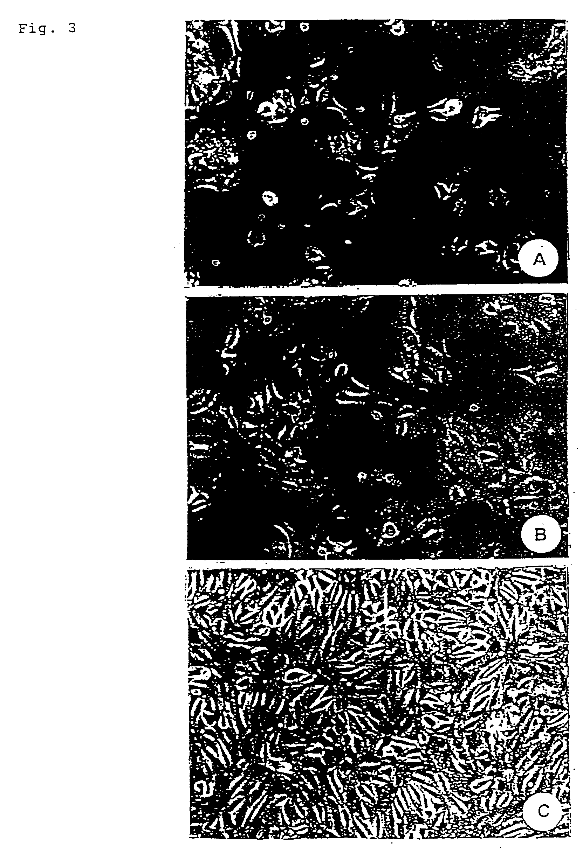

Poly-D-Lysine beschichteten Oberflächen. Am 9. Tag nach der Einsaat waren die Zellen konfluent gewachsen (Fig. 2 und 3). Die Verdoppelungszeit der Vero-Zellen auf PVF im proteinfreien Medium wurde nach 48 Stunden erreicht, in DMEM-F12, 10% FBS enthaltendem Medium nach 28 Stunden. Die Sättigungsdichte der Zellen unter Verwendung von serum-haltigem Medium war nur ungefähr 17% höher als jene unter Verwendung von protein-freiem Medium auf PVF (Fig. 2).

In Tabelle 3 ist die Wirkung der verschiedenen Oberflächen auf die Adhäsion von Vero-Zellen in dem proteinfreien Medium dargestellt. Die an den Oberflächen durch Poly-D-Lysine und PVF festgehaltenen Zellen verbreiteten sich schneller als an den PS-TC enthaltenden Oberflächen. Dies konnte 30 Minuten nach der Beimpfung klar gesehen werden. Die Zahl der an den PS-B-Oberflächen gebundenen Zellen entsprach der Zahl der Zellen an PS-TC; darüber hinaus wurde eine beginnende Ausbreitung beobachtet.

Die in Tabelle 4 dargestellten Ergebnisse zeigen auf, daß die Ausbreitung der Vero-Zellen in dem protein-freien Medium stark abhängig von der Zahl der eingesäten Zellen ist, wobei nur im Fall einer ursprünglichen Vero-Zellen-Dichte von über $0.2 \times 10^4$ Zellen/cm$^2$ sich die Zahl der Zellen steigern konnte. Eine geringe beginnende Ausbreitung von Vero-Zellen zeigte sich an PS und Poly-D-Lysine, aber nur bei einer Einsaat mit einer anfänglichen Dichte von jeweils $2 \times 10^4$ Zellen/cm$^2$ und $1 \times 10^4$ Zellen/cm$^2$.

Tabelle 5 stellt das Wachstum der Vero-Zellen in proteinfreiem Medium DMEM-F12 an PVF-beschichteten Oberflächen in Abhängigkeit verschiedener Formvar-Konzentrationen dar. Die Sättigungsdichten auf PVF-Oberflächen auf der Basis von Formvar-Lösungen mit Konzentrationen in den Bereichen von 0.5% bis 2% unterscheiden sich nicht wesentlich. Demgegenüber wurde eine signifikante Abnahme der Sättigungsdichte der Zellen im Fall von PVF mittels 0.1%iger Formvar-Lösung beobachtet.

Aus Tabelle 6 kann man entnehmen, daß PVF-enthaltende Oberflächen mindestens bis zu 3 Monate nach ihrer Vorbereitung ohne Abnahme der Wachstumsrate der Vero-Zellen in dem proteinfreien Medium verwendet werden können.

Die Messung der negativen Oberflächen-Ladungsdichte weißt signifikante Unterschiede zwischen PS-TC- und PVF-behandelten Oberflächen auf. Andererseits hat man keine wesentlichen Unterschiede zwischen PVF und PS-B beobachtet. Der Bereich der Oberflächen-Ladungsdichte betrug $4-7 \times 10^{14}$ per cm$^2$ für PS-TC, $0.3-0.7 \times 10^{14}$ per cm$^2$ für PS-B und $0.4-0.8 \times 10^{14}$ per cm$^2$ für PVF.

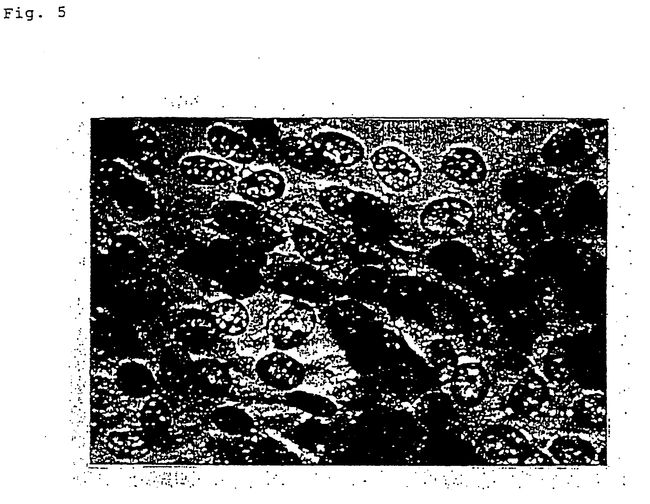

Die Sub-Kultivierung von Vero-Zellen in proteinfreiem Medium auf PVF konnte durchgeführt werden, ohne eine Abnahme der Wachstumsrate in aufeinanderfolgenden Durchführungen festzustellen, wobei bis zu fünfzig Sub-Kultivierungen, die zusammen 164 Populationsverdoppelungen erreichten, durchgeführt wurden. Das morphologische Erscheinungsbild der in dem proteinfreien Medium etablierten Vero-Zellen unterscheidet sich nicht von jenem der Zellen in entsprechendem serumhaltigen Medium (Fig. 5). Darüber hinaus stellt Fig. 5 dar, daß keine Hintergrundfärbung nach der Färbung mit May-Grünwald-Giemsa auftritt.

Die PVF-Schicht in obigem Beispiel besitzt optische Eigenschaften, die jener der PS-Kulturkolben ähnlich sind. Darüber hinaus ist nach Färbung mit May-Grünwald-Giemsa keine Hintergrundfärbung von PVF zu beobachten. Wir haben mehrere PVF-Zubereitungen getestet, die im Anteil an Hydroxy und Acetat unterschiedlich waren, jedoch ähnliche Wirkungen in der Ausbreitung der Vero-Zellen aufzeigten. Durch die Eigenschaften und die geringen Kosten von PVF eignet es sich als Kulturoberfläche für Vero-Zellen und auch eventuell für LLC-MK2- und MDCK-Zellen, deren Ausbreitung in proteinfreiem Medium an PVF ebenfalls gesteigert wurde. Da Vero-Zellen oft in der Produktion von Virusimpfstoff oder anderen biologisch aktiven Substanzen Verwendung findet, können z.B. PVF-beschichtete Microcarrier der Herstellung von biologischen Substanzen dienen.

In dieser Untersuchung verwendeten wir relativ einfache proteinfreie Medien für die verschiedenen Zellinien. Denkbar ist, daß eine Verbesserung der proteinfreien Medien ein Wachstum der Zellen an PVF noch steigern würde. Es muß berücksichtigt werden, daß ein erfolgreiches Wachstum der verschiedenen Zellen in proteinfreien Medien von verschiedenen Faktoren einschließlich Reinheit des Wassers, chemisch reine Glas- und Kunststoffware und beste Qualität der Chemikalien abhängt [6, 16, 17, 29]. Wir ergänzten das proteinfreie Medium mit Glycin-L-Glutamine anstatt mit L-Glutamine [30] und mit L-Ascorbinsäure-2-Phosphat [31]. Es ist zu vermuten, daß die Verwendung dieser stabilen Substanzen die Verbreitung der verschiedenen Zellen in proteinfreien Medien erhöht.

Wie oben in dem Beispiel bereits hingewiesen, wachsen nicht alle Zelltypen auf PVF so hervorragend wie die Vero-Zellen. Die Identifizierung von passenden Zelltypen kann aber ganz einfach durch Anwendung der Prozeduren analog des obigen Beispiels durchgeführt werden.

TABELLE 1

| Substanzen mit proteinfreiem DMEM-F12 | | |
|---|---|---|
| Substanz | Konzentration im Medium mg/l | Quelle* |
| $CoCL_2.6H_2O$ | 0.0002 | S |
| $CuSO_4.5H_2O$ | 0.00025 | S |
| $MnSO_4.7H_2O$ | 0.000024 | SE |
| $Na_2SeO_3$ | 0.0005 | S |
| $Na_2SiO_3.9H_2O$ | 0.0065 | I |
| $NH_4VO_3$ | 0.00006 | SE |
| $NiCl_2.6H_2O$ | 0.000012 | SE |
| $(NH_4)_6Mo_7O_{24}.H_2O$ | 0.00012 | SE |
| $SnCl_2.2H_2O$ | 0.00001 | S |
| L-Ascorbinnsäure 2-Phosphat-Magnesiumsalz | 28.95 | W |
| Glycyl-L-Glutamin | 800.00 | PK |

\* S  =  Sigma;

SE  =  Serva (Heidelberg, BRD);

I  =  ICN Biochemicals (Mechenheim, BRD);

W  =  Wako Pure Chemical Industrie, Ltd. (Osaka, Japan);

PK  =  Pfrimmer - Kabi (Erlangen, BRD)

**TABELLE 2**

Wirkung von verschiedenen Oberflächen bei Sättigungsdichte auf zehn Säugetier-Zellinien in proteinfreiem Medium

| Zellinie | Herkunft Gewebe | Art | Zellenanzahl per $cm^2$ x $10^{-4}$ PS-TC | Poly-D-Lysine | PVF |
|---|---|---|---|---|---|
| NTCT Klon 929 | Unterhaut- und Fettgew. | Maus | 4.6±0.18(+) | 7.3±9.46(++) | 4.2±0.27(+) |
| 3T6 | Embryo | Maus | 8.1±0.45(++) | 9.3±0.52(+++) | 2.5±0.19(+) |
| BHK | Niere | Hamster | 4.5±0.31(+) | 5.1±0.39(+) | 4.9±0.29(+) |
| CHO-K1 | Eierstock | chines. Hamster | 5.1±0.38(+) | 7.2±0.38(++) | 5.7±0.32(+) |
| PK 15 | Niere | Schwein | 6.9±0.49(++) | 7.9±0.43(++) | 7.4±0.45(++) |
| MDCK | Niere | Hund | 4.9±0.31(+) | 8.3±0.48(++) | 10.4±1.62(++) |
| Vero | Niere | Affe | 5.6±0.29(+) | 8.1±0.41(++) | 19.2±2.10(++++) |
| LLC-MK2 | Niere | Affe | 5.2±0.31(+) | 9.8±0.51(+++) | 8.7±0.51(++) |
| A431 | Oberhaut-krebs | Mensch | 5.9±0.49(+) | 10.1±1.70(+++) | 6.3±0.51(+) |
| HeLa | Gebärmutter-halskrebs | Mensch | 6.6±0.43(++) | 8.1±0.43(++) | 5.9±0.39(+) |

Die PVF-Oberfläche wurde aus einer 1%igen Formvar-Lösung hergestellt. Die Menge entspricht vier Schalen ±SEM. Die Zellen erreichten maximal 25%ige Konfluenz (+), 50%ige Konfluenz (++), 75%ige Konfluenz (+++) oder Konfluente (++++) Zellendichte.

TABELLE 3

| Wirkung der verschiedenen Oberflächen auf die Adhäsion der Vero-Zellen im proteinfreien Medium | | | | |
|---|---|---|---|---|
| Zeit nach Beimpfung (Stunden) | Zellenanzahl/$cm^2$ x $10^{-4}$ | | | |
| | PS-B | PS-TC | Poly-D-Lysine | PVF |
| 30 | 1.31±0.21 | 1.49±0.23 | 1.81±0.25 | 1.74±0.19 |
| 60 | 1.56±0.19 | 1.61±0.21 | 1.78±0.21 | 1.86±0.23 |
| 90 | 1.61±0.22 | 1.68±0.26 | 1.83±0.18 | 1.85±0.25 |

Die PVF-Oberfläche wurde aus einer 1%igen Formvar-Lösung hergestellt. Die Menge entspricht vier Schalen ±SEM.

# EP 0 531 562 A1

TABELLE 4

| Wirkung der Ausgangszelldichte auf die Ausbreitung der Vero-Zellen an der PVF-Oberfläche in proteinfreiem Medium | | | |
|---|---|---|---|
| Ausgangs-Zelldichte per $cm^2$ x $10^{-4}$ | Zelldichte per $cm^2$ x $10^{-4}$ nach Abschluß | | |
| | PS-TC | Poly-D-Lysine | PVF |
| 2 | 5.5±0.29 | 8.9±0.45 | 18.9±2.9 |
| 1 | 2.2±0.21 | 4.6±0.31 | 14.2±2.1 |
| 0.5 | 0 | 0 | 6.9±0.4 |
| 0.2 | 0 | 0 | 3.8±0.3 |
| 0.1 | 0 | 0 | 0 |

Die PVF-Oberfläche wurde aus einer 1%igen Formvar-Lösung hergestellt. Die Menge entspricht vier Schalen ±SEM.

TABELLE 5

| Wirkung der PVF-Oberfläche von Formvar-Lösungen verschiedener Konzentrationen auf die Sättigungsdichte von Vero-Zellen im proteinfreiem Medium | |
|---|---|
| PFV-Konzentration in % | Zellenanzahl per $cm^2$ x $10^{-4}$ |
| 0.10 | 12.5±2.5 |
| 0.50 | 18.6±3.1 |
| 1.00 | 18.3±2.8 |
| 2.00 | 18.9±2.4 |

Die Menge entspricht vier Schalen ±SEM. Die Zellen wurden 9 Tage nach Beimpfung ausgezählt.

16. Price, F.M., and Sanford, K.K. (1976) Procedure 10311. TCA Manual 2, 379-382

17. Cinatl, J. jr., Türk, M., Cinatl, J., Ahn, J.W., Rabenau H., and Doerr, H.W. (1990) In Vitro Cell. Dev. Biol. 26, 841-842

18. Maroudas, N.G. (1975) J. Theor. Biol. 49, 417-424

19. Maroudas, N.G. (1976) J. Cell. Physiol. 90, 511-520

20. Chen, T.R. (1977) Exp. Cell Res. 104, 255-262

21. McKeehan, W.L., McKeehan, K.A., Hammond, S.L., and Ham, R.G. (1977) In Vitro Cell. Dev. Biol. 13, 399-416

22. Schaeffer., W.I. (1984) In Vitro Cell. Dev. Biol. 20, 19-24

23. Kovar, J. (1989) In Vitro Cell Dev. Biol. 25, 395-396

24. Rappaport, C., Poole, J.P., and Rappaport, H.P. (1960) Exp. Cell Res 20, 465-510

25. Barski, G., and Maurin, J. (1948) Annal. Inst. Pasteur 74, 312-322

26. Furusawa, K., Suda, Y., and Tsuda, K. (1980) Kenkyu Hokoku-Sen'i Kobunshi Zairyo Kenkyosho 124, 17-21

27. Craig, A.M., Smith, J.H., and Denhardt, D.T (1989) J. Biol. Chem. 264, 9682-9689

28. Nordby, A., Tredt, K.E., Halgunset, J., Kopstad, G., and Haugen, O.A., (1989) Invest. Radiol. 24, 703-710

29. Cinatl, J. jr., Cinatl, J., Rabenau, H., and Doerr, H.W. (1989) J. Tissue Cult. Methods 12, 67-72

30. Roth, E., Ollenschlager, G., Hamilton, G., Simmel, A., Langer, K., Fekl, W., and Jakesz, R. (1988) In Vitro Cell. Dev. Biol. 24, 696-698

31. Hata, R., and Senoo, H. (1989) J. Cell. Physiol. 138, 8-16

References:

1. Evans, V.J., Bryant, C.J., Fiaramonti, M.C, McQuilkin, W.T., Sanford, K.K., and Earle, W.R. (1956) Cancer Res. 16, 77-86

2. Evans, V.J., Kerr, H.A., McQuilkin, W.T., Earle, W.R., and Hull, R.N. (1959) Am. J. Hyg. 70, 297-302

3. Evans, V.J., LaRock, J.F., Yosida, T.H., and Pottre, M. (1963) Exp. Cell Res. 32, 212-217

4. Cinatl, J., and Vesely, P. (1967) Folia Biologica 13, 61-67

5. Takoaka, T., and Katsuta, H. (1971) Exp. Cell Res. 67, 295-304

6. Sanford, K.K., and Evans, V.J. (1982) J. Natl. Cancer Ins. 68, 895-913

7. Okabe, T., Fujisawa, M., and Takaku, F. (1984) Proc. Natl. Acad. Sci. USA 81, 453-455

8. Yamaguchi, N., Morioka, H., Ohkura, H., Hirohashi, S., and Kawai, K. (1985) J. Natl. Cancer Inst. 75, 29-35

9. Yamaguchi, N., and Kawai, K. (1986) Cancer Res. 46, 5353-5359

10. Kovar, J., and Franek, F. (1987) Biotechnol. Lett. 9 ), 259-264

11. Cinatl, J. jr., Cinatl, J., Gerein, V., Kornhuber, B., and Doerr, H.W. (1988) J. Biol. Stand. 16, 249-257

12. Hill, M., Hillova, J., Mariage-Samson, R., Fasciotto, B., and Krsmanovic, V. (1989) In Vitro Cell. Dev. Biol. 25, 49-56

13. Rikimaru, K., Toda, H., Tachikawa, N., Kamata, N., and Enomoto, S. (1990) In Vitro Cell. Dev. Biol. 26, 849-856

14. Fike, R., Pfohl, J., Jayme, D., and Weiss, S. (1990) In Vitro Cell. Dev. Biol. 26, 54A

15. Darfler, F.J. (1990) In Vitro Cell. Dev. Biol. 26, 769-778

**Patentansprüche**

1.  Verfahren zur Kultivierung von adhärenten Zellen in proteinfreiem Medium, dadurch gekennzeichnet, daß man Kulturoberflächen aus Polyvinylformal und/oder Polyvinylbutyral verwendet.

2.  Verfahren nach Anspruch 1, dadurch gekennzeichnet, daß man Polyvinylformal als Kulturoberfläche verwendet.

3.  Verfahren nach Anspruch 1 oder 2, dadurch gekennzeichnet, daß die Zellen als kontinuierliche Monolayer kultiviert werden.

4.  Verfahren nach Anspruch 1, 2 oder 3, dadurch gekennzeichnet, daß die Zellen Vero-Zellen sind.

5.  Verfahren nach Anspruch 4, dadurch gekennzeichnet, daß die Zellen mit Viren infiziert werden oder auf natürliche oder rekombinierte Weise biologische Substanzen produzieren.

6.  Verfahren nach Anspruch 4 oder 5, dadurch gekennzeichnet, daß die Zellen ursprünglich bei einer Zelldichte von mindestens $2.0 \times 10^4$ Zellen/cm$^2$ auf die Oberfläche geimpft werden.

7.  Ein Bioreaktor zur Kultivierung der Zellen in proteinfreiem Medium, dadurch gekennzeichnet, daß er eine Kulturoberfläche aus PVF enthält.

8.  Ein Bioreaktor nach Anspruch 7, dadurch gekennzeichnet, daß die Oberfläche von PVF und/oder PVB aus einer Wand des Reaktors und/oder aus der Fläche der Füllungskörper besteht.

9.  Die Verwendung eines Bioreaktors nach Anspruch 7 oder 8 zur Kultivierung von adhärenten Zellen in proteinfreiem Medium.

10. Ein Füllungskörper mit einer Oberfläche aus PVF und/oder PVB zur Verwendung in einem Verfahren zur Kultivierung von adhärenten Zellen in proteinfreiem Medium.

Fig. 1

PV Acetate

PV Alkohol

PV Formal

Fig. 2

Fig. 3

Fig. 4

Fig. 5

Europäisches
Patentamt

**EUROPÄISCHER RECHERCHENBERICHT**

Nummer der Anmeldung

EP    91 11 5336

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 400 063 (INSTITUT PASTEUR) <br> * das ganze Dokument * | 7,8,10 | C12N5/00 |
| Y | | 1-6,9 | |
| | --- | | |
| Y | JOURNAL OF CELL SCIENCE <br> Bd. 86, 1986, LONDON, GB <br> Seiten 9 - 24; <br> A.S. CURTIS ET AL.: 'SUBSTRATE HYDROXYLATION AND CELL ADHESION' <br> * das ganze Dokument * | 1-6,9 | |
| | --- | | |
| A | BIOLOGICALS <br> Bd. 19, Nr. 2, April 1991, GENEVA, CH <br> Seiten 87 - 92; <br> J. CINATL JR. ET AL.: 'QUALITY CONTROL TESTING OF SURFACES FOR MAMMALIAN CELL CULTURE USING CELLS PROPAGATED IN A PROTEIN-FREE MEDIUM.' <br> * das ganze Dokument * | 1-10 | |
| | ----- | | |

| RECHERCHIERTE SACHGEBIETE (Int. Cl.5) |
|---|
| C12N |

Der vorliegende Recherchenbericht wurde für alle Patentansprüche erstellt

| Recherchenort | Abschlußdatum der Recherche | Prüfer |
|---|---|---|
| DEN HAAG | 08 MAI 1992 | RYCKEBOSCH A.O. |